# EUROPÄISCHE PATENTSCHRIFT

(11) **EP 1 096 906 B1**
(45) Veröffentlichungstag und Bekanntmachung des Hinweises auf die Patenterteilung: **01.10.2003**
(21) Anmeldenummer: 99942793.3
(22) Anmeldetag: 20.07.1999
(51) Int. Cl.: A61F 2/42

(54) **FINGERGELENKPROTHESE**
FINGER JOINT PROSTHESIS
PROTHESE D'ARTICULATION DU DOIGT

(30) Priorität: 21.07.1998 DE 29813030 U
(43) Veröffentlichungstag der Anmeldung: 09.05.2001
(73) Patentinhaber: Ranft, Christoph, 24106 Kiel (DE)
(72) Erfinder: Ranft, Christoph, 24106 Kiel (DE)
(74) Vertreter: Hano, Christian, Dipl.-Ing.
(86) Internationale Anmeldenummer: EP9905176
(87) Internationale Veröffentlichungsnummer: WO00004850

(56) Entgegenhaltungen:
- WO-A-90/11739
- US-A- 3 638 243
- US-A- 3 651 521
- US-A- 3 869 729
- US-A- 5 133 761

## Beschreibung

Die Erfindung betrifft eine Fingergelenkprothese nach dem Oberbegriff des Patentanspruchs 1.

Funktionsstörungen von Fingermittelgelenken entstehen häufig aufgrund einer vererbten degenerativen Erkrankung, der Gelenkarthrose. In diesem Fall führt die Erkrankung zum "Verschleiß" des Gelenkknorpels und in der Folge zu entsprechenden Fehlbelastungen mit kompletten Veränderungen der Gelenkflächen, die eine schmerzhafte Einschränkung oder sogar Aufhebung der Gelenkfunktion zur Folge haben.

Funktionsstörungen der Fingermittelgelenke können jedoch auch aufgrund einer Verletzung, entweder durch Luxation des Gelenkes oder durch Gelenkbruch entstehen. Eine primäre Behandlung eines Gelenkbruchs führt in der Regel zu einer sogenannten "posttraumatischen Arthrose" des Gelenkes, was ebenfalls zu einer schmerzhaften Funktionseinschränkung oder Funktionsaufhebung des Fingermittelgelenks führt.

Ein Weg zur Beseitigung der oben genannten Funktionsstörungen ist die Versteifung des Gelenkes in einer funktionsgünstigen Stellung. Dieser Weg führt zwar zur Schmerzfreiheit, bedeutet aber die vollständige Funktionsunfähigkeit des Gelenkes.

Da gerade die Fingermittelgelenke für eine gute Greiffunktion der Hand erforderlich sind, wurden Fingergelenkprothesen entwickelt, die das Fingermittelgelenk ersetzen können. Eine solche Fingergelenkprothese ist beispielsweise in dem Prospekt "DIGITOS Die modulare Fingergelenks-Totalendoprothese" der Firma OSTEO AG beschrieben. Diese Fingergelenkprothese umfaßt ein U-förmiges Gelenkelement, an dem ein im Querschnitt rechteckiger Verankerungsschaft vorgesehen ist. Ein scheibenförmiges Gelenkelement mit einem ebenfalls im Querschnitt rechteckigen Verankerungsschaft greift so zwischen die beiden Schenkel des U-förmigen Gelenkelements ein, daß Durchgangsbohrungen in den Schenkeln des U-förmigen Gelenkelements mit einer Öffnung in dem scheibenförmigen Gelenkelement fluchten.

Für die Implantation der bekannten Fingergelenkprothese wird für einen ulnaren Zugang das innere Seitenband an dem ersten Fingerglied abgelöst. Das Gelenk wird dann seitlich luxiert und ein Teil der Palmarplatte abgelöst. Anschließend werden der Kopf des ersten Fingerglieds und die Basis des zweiten Fingerglieds so resiziert, daß zwischen den beiden Fingergliedern ein vorher bestimmter Abstand vorhanden ist. Nun wird in jedes Fingerglied ein sich entlang seiner Mittelachse erstreckender rechteckiger Raum geraspelt. In diesen rechteckigen Raum wird eine Schaftführung einzementiert. Anschließend werden die Gelenkelemente einzeln mit ihren Verankerungsschäften in die Schaftführungen eingesetzt, die Fingerglieder zurückgebogen und das erste und zweite Gelenkelement durch Einsetzen einer Achse in die fluchtenden Öffnungen gelenkig miteinander verbunden.

Da bei der Implantation der bekannten Fingergelenkprothese das Fingermittelgelenk seitlich luxiert werden muß, werden der Strecksehnenapparat, die beiden Beugesehnen sowie die Seitenbänder des Gelenkes irritiert, was zu einer späteren Beeinträchtigung der Funktionstätigkeit führt. Darüber hinaus wird eine große Menge an Knochensubstanz für die Implantation der Fingergelenkprothese geopfert, da eine Schaftführung in Richtung der Mittelachse der Fingergelenke eingeführt werden muß.

Aus der WO 90/11739 ist eine Fingergelenkprothese mit einem Gelenkkörper bekannt, der ein erstes und ein zweites Gelenkelement aufweist, die schwenkbar miteinander verbunden sind und von deren Außenumfangsfläche sich jeweils ein schmales Verankerungselement zur Befestigung an einem proximalen bzw. distalen Fingerglied tangential erstreckt. Das zweite Gelenkelement weist eine gabelförmige Ausparung auf, in der das erste Gelenkelement aufgenommen ist. Zur Implantation dieser Fingergelenkprothese wird der Gelenkkopf von dem Ende des proximalen Fingerglieds entfernt, damit der Gelenkkörper eingesetzt werden kann. Anschließend wird die Fingergelenkprothese so eingesetzt, dass die Verankerungsleisten außen auf den Fingergliedern aufliegen. Dann werden die Verankerungsleisten mit den Fingergliedern verschraubt. Die Verankerungsleisten sind daher nach der Operation von außen sichtbar.

Der Erfindung lag die Aufgabe zugrunde, mit konstruktiv einfachen Mitteln eine stabile und leicht implantierbare Fingergelenkprothese zu schaffen, die nach deren Implantation die wesentlichen Funktionen des Fingergelenks weitgehend erhalten werden.

Diese Aufgabe wird durch eine Fingergelenkprothese mit den Merkmalen des Patentanspruchs 1 gelöst. Vorteilhafte Weiterbildungen der erfindungsgemäßen Fingergelenkprothese sind Gegenstand der Patentansprüche 2 bis 5.

Da bei der erfindungsgemäßen Fingergelenkprothese der Gelenkkörper im wesentlichen zylindrisch ist, wenn sich die beiden Gelenke in einer Implantierstellung befinden, kann der Gelenkkörper von radialer Seite in eine durch einen Rundfräser vorbereite Bohrung eingesetzt werden. Die Verankerungselemente können in schmale Schlitze eingesetzt werden, die in Längsrichtung der Fingerglieder verlaufen und von radialer Seite her eingefräst werden. Da für den Einbau des erfindungsgemäßen Fingergelenkes keine seitliche Luxation des Fingergelenkes erforderlich ist, wird die Funktionsfähigkeit der sensiblen Strukturen wie Gelenkkapsel, Strecksehnenapparat und Beugesehnen durch die Implantation kaum beeinträchtigt. Außerdem ist die Fingergelenkprothese nach der Implantation nicht sichtbar. Schließlich muß für die Implantation der Fingergelenkprothese wenig Knochensubstanz geopfert werden, da nur schmale Schlitze für die Aufnahme der Verankerungsleisten erforderlich sind.

Ein weiterer Vorteil der erfindungsgemäßen Fingergelenkprothese liegt darin, daß die Fingergelenkprothese im zusammengebauten Zustand implantiert werden kann. Die Implantation ist darüber hinaus mit einem sehr einfach zu handhabenden Instrumentarium möglich.

Die Elemente der erfindungsgemäßen Fingergelenkprothese bestehen bevorzugt aus Titan, da das Einwachsen von Knochen in die poröse Oberfläche dieses Materials eine hohe und dauerhafte Implantatstabilität bewirkt.

Es ist außerdem möglich, die Verankerungsleisten mit Hilfe von Titanschrauben an den Fingergliedern zu fixieren, die durch Ösen hindurchgehen, die in Seitenwänden der Verankerungselemente vorgesehen sind.

Ein Ausführungsbeispiel der Erfindung wird nachstehend an Hand der Zeichnung näher erläutert. Es zeigen:
- Fig.1: schematisch eine radiale Ansicht eines Fingermittelgelenks, wobei der Ort für eine Implantation einer Fingergelenkprothese angedeutet ist;
- Fig.2: eine schematische Draufsicht auf eine Fingergelenkprothese;
- Fig. 3: eine schematische Seitenansicht der Fingergelenkprothese von Fig. 2;
- Fig. 4: eine perspektivische Ansicht eines zweiten Gelenkelements der Fingergelenkprothese von Fig. 2;
- Fig. 5: eine perspektivische Ansicht eines ersten Gelenkelements der Fingergelenkprothese von Fig. 2;
- Fig.6: den Mittelsteg eines T-förmigen Achselements;
- Fig.7: den Quersteg des T-förmigen Achselements;
- Fig.8: in einer teilweisen Querschnittsansicht die Fingergelenkprothese von Fig. 2 in implantiertem Zustand, wobei sich die Fingergelenkprothese in einer Implantierstellung befindet;
- Fig. 9: eine Draufsicht auf die in Fig. 8 gezeigte implantierte Fingergelenkprothese.

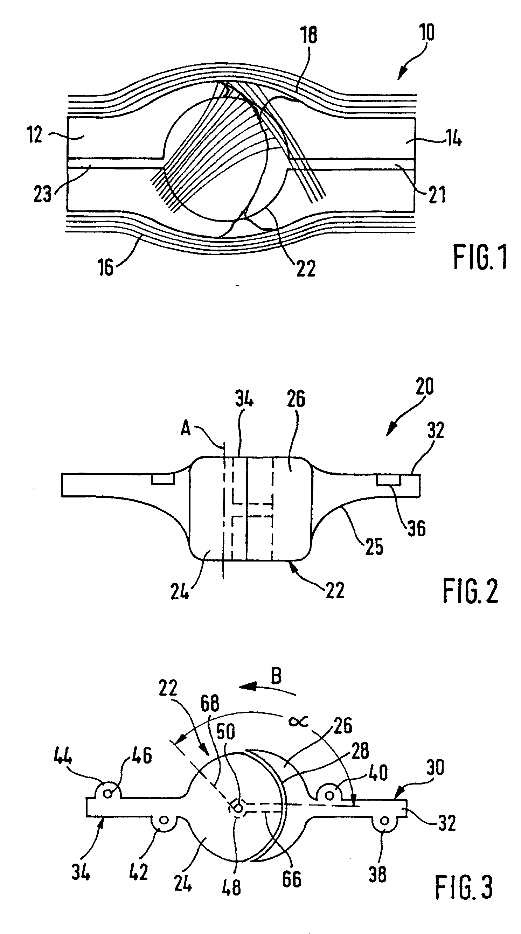

Fig. 1 zeigt die radiale Seite eines Fingermittelgelenks 10 mit dem proximalen, d.h. körpernahen Fingerglied 12 und dem distalen, d. h. körperfernen Fingerglied 14. Außerdem sind unterhalb des Fingermittelgliedes die Strecksehne 16 und oberhalb des Fingermittelgliedes die Beugesehnen 18 gezeigt.

Die in den Fig. 2 und 3 gezeigte Fingergelenkprothese 20 dient zur radialen Implantierung in das in Fig. 1 gezeigte Fingergelenk 10 an dem Ort des Scharniergelenks, der durch den Kreis 22 angedeutet ist.

Die in Fig. 2 gezeigte Fingergelenkprothese 20 weist einen Gelenkkörper 22 auf, der ein erstes Gelenkelement 24 und ein zweites Gelenkelement 26 umfaßt, die gelenkig so miteinander verbunden sind, daß das zweite Gelenkelement 26 bezüglich des ersten Gelenkelements 24 um eine in Fig. 2 vertikale Achse A verschwenkt werden kann.

Die Fig. 2 und 3 zeigen die Fingergelenkprothese in ihrer Draufsicht bzw. Seitenansicht in Implantierstellung. In dieser Implantierstellung hat der Gelenkkörper 22 eine im wesentlichen zylindrische Form. Wie es in Fig. 3 gezeigt ist, ist das zweite Gelenkelement 26 im Querschnitt sichelförmig ausgebildet. Das erste Gelenkelement 24 ist so ausgebildet, daß es mit seiner Außenumfangsfläche in den konkaven Abschnitt 28 des sichelförmigen Gelenkelements 26 so eingreift, daß ein geringfügiger Spalt zwischen der Außenumfangsfläche des ersten Gelenkelements 24 und der konkaven Fläche des zweiten Gelenkelements 26 vorhanden ist. Das erste Gelenkelement 26 und das zweite Gelenkelement 24 bilden somit in der in den Fig. 2 und 3 gezeigten Implantierstellung den insgesamt zylindrischen Gelenkkörper 22.

Von der Mitte (Fig.3) der Außenumfangsfläche des zweiten Gelenkelements 26 erstreckt sich eine in Axialrichtung des Gelenkkörpers 22 gesehen (Ansicht von Fig.3) schmale Verankerungsleiste 30 radial nach außen. Die in Fig. 2 obere Längswand 32 ist im wesentlichen bündig mit der oberen Stirnseite 34 des Gelenkkörpers 22. Die Höhe der Verankerungsleiste 30 beträgt am äußeren Ende ca. 1/4 der Höhe des Gelenkkörpers 22 und nimmt in radialer Richtung nach innen zu. Am Übergang der Verankerungsleiste 30 zu dem Gelenkkörper 22 hat das Verankerungselement 30 die Höhe des Gelenkkörpers 22, wobei die untere Längswand 25 ungefähr parabelförmig verläuft.

Eine weitere Verankerungleiste 34 erstreckt sich von der Außenumfangsfläche des ersten Gelenkelements 24 ebenfalls radial nach außen, wobei die beiden Verankerungsleisten in der Implantierstellung 30, 34 in einer geraden Linie angeordnet sind. An jeder Seitenwand 36 der Verankerungselemente 30, 34 ist angrenzend an die Längswand 32 eine Befestigungsöse 38, 40, 42 bzw. 44 angeordnet. Jede dieser Befestigungsösen weist eine Öffnung 46 auf, deren Mittelachse etwa parallel zur Mittelachse des Gelenkkörpers 22 verläuft. Bei dem ersten Gelenkelement 24 ist eine Öse 42 an der in Fig. 3 unteren Seitenwand der Verankerungsleiste 34 in der Nähe der Außenumfangsfläche des ersten Gelenkelements 24 vorgesehen, während die Öse 44 an der oberen Seitenwand angrenzend an das radial äußere Ende der Verankerungsleiste vorgesehen ist. Die an der oberen Seitenwand des Verankerungselements 30 des zweiten Gelenkelements 26 vorgesehene Befestigungsöse 40 ist angrenzend an die Außenumfangsfläche des zweiten Gelenkelements 26 angeordnet, während die an der unteren Seitenwand der Verankerungsleiste 30 vorgesehene Öse in der Nähe des radial äußeren Endes der Verankerungsleiste 30 vorgesehen ist.

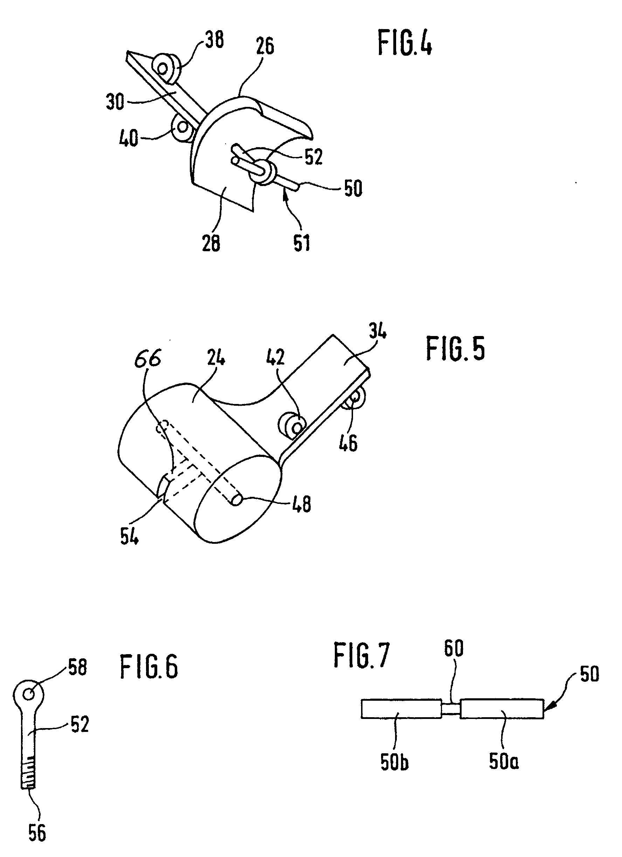

Wie es in den Fig. 4 und 5 gezeigt ist, erstreckt sich von dem Zentrum der konkaven Fläche 28 des zweiten Gelenkelements 26 ein Mittelsteg 52 eines T-förmigen Achselements 51 in radialer Richtung. Der Mittelsteg 52 ist mittels eines Gewindes 56 (Fig. 6) in eine entsprechende Gewindebohrung (nicht gezeigt) in der konkaven Fläche 28 verschraubt. An seinem freien Ende weist der Mittelsteg 52 ein Auge 58 auf. Durch das Auge 58 ist in zusammengebautem Zustand der Fingergelenkprothese eine Achse 50 durchgeführt, deren Außendurchmesser insgesamt dem Durchmesser des Auges 58 entspricht. Die Achse 50 weist in ihrer Längsmitte eine Einschnürung 60 mit verringertem Durchmesser auf (Fig.7), der kleiner als der Durchmesser des Auges 58 ist.

Die Achse 50 (Fig.7) weist ein erstes Element 50a auf, das einen Bereich größeren Durchmessers und den Bereich der Einschnürung (60) umfaßt, wobei sich von der Einschnürung aus in Längsrichtung ein Gewindeabschnitt (nicht gezeigt) erstreckt, der in eine Gewindebohrung (nicht gezeigt) eines zweiten Elements (50b) mit größerem Durchmesser eingeschraubt ist.
Im montierten Zustand der Fingergelenkprothese ist die Achse 50 in eine durch das erste Gelenkelement 24 hindurchgehende Achsbohrung 48 eingesetzt, die entlang der Mittelachse des zylindrischen Gelenkkörpers 22 verläuft. Der Innendurchmesser der Achsbohrung 48 entspricht im wesentlichen dem Außendurchmesser der Achse 50.

Auf mittlerer Höhe des ersten Gelenkelements 24 ist in der Außenumfangsfläche ein Radialschlitz 54 vorgesehen (Fig.5), der durch zwei radiale Innenwände 66 und 68 begrenzt ist, die in einem Winkel α (Fig. 3) von ca. 135° zueinander angeordnet sind. Der Schlitz 54 mündet in dem Scheitel der Innenwände 66, 68 in die Achsbohrung 48. In der in Fig. 3 gezeigten Implantierstellung der Fingergelenkprothese verläuft die eine radiale Innenwand 66 des Schlitzes 54 in gleicher Richtung wie die Verankerungsleiste 30. Die andere radiale Innenwand 68 ist bezüglich der einen Innenwand 66 in Drehrichtung (Pfeil B) um ca. 135° versetzt.

Zur Montage der Fingergelenkprothese wird der an dem zweiten Gelenkelement 26 befestigte Mittelsteg 52 so in den Schlitz 54 eingeführt, daß das Auge 58 mit der Achsbohrung 48 fluchtet. Anschließend werden das erste Element (50a und das zweite Element 50b der Achse 50 so in der Achsbohrung 48 zusammengeschraubt, daß die Einschnürung 60 auf der Höhe des Schlitzes 54 liegt, und somit ein geringes Spiel zwischen dem Auge 58 und der Achse 50 besteht.

In der in Fig. 3 gezeigten Implantierstellung liegt der Mittelsteg 52 an der einen radialen Innenwand 66 an. Die radiale Innenwand 66 bildet somit einen Anschlag, der die Drehung des zweiten Gelenkelements 26 begrenzt.

Bei einer Drehung des zweiten Gelenkelements 26 bezüglich des ersten Gelenkelements 24 in Richtung des Pfeiles B (Fig. 3) wird das zweite Gelenkelement 26 um die Achse 50 verschwenkt und gleitet dabei entlang der Außenumfangsfläche des ersten Gelenkelements 24. Das Spiel zwischen dem Auge 58 und der Einschnürung 60 ermöglicht es, die Außenumfangsfläche des ersten Gelenkelements 24 so auszugestalten, daß das an ihr gleitende zweite Gelenkelement 26 keine reine Kreisbewegung durchführt. Vorzugsweise ist die Form des ersten Gelenkelements 24 so bestimmt, daß die Spitze des Fingergrundgliedes bei einer Drehung des zweiten Gelenkelements 26 in Richtung des Pfeiles B einer Fibonacci-Kurve, d.h. einer gleichwinkligen oder logarithmischen Spirale folgt.

Alternativ kann die Einschnürung 60 in der Achse 50 weggelassen werden, wenn die Achsbohrung 48 als Langlochbohrung ausgeführt ist.

Zur Vorbereitung der Implantation der Fingergelenkprothese wird nach Ablösung des entsprechenden Seitenbandes mittels eines Rundfräsers von der radialen Seite her eine Bohrung an dem Ort gefräst, der durch den Kreis 22 in Fig. 1 angedeutet ist. Der Bohrmittelpunkt entspricht dem Drehmittelpunkt der Fingerglieder 12, 14 zu Beginn der Verschwenkung des distalen Fingergliedes 14 aus der Streckstellung in die Beugestellung. Der Innendurchmesser und die Höhe der Bohrungen entsprechen dem Außendurchmesser und der Höhe des Gelenkkörpers 22. Nach dem Fräsen der Bohrung werden mit einem Schlitzfräser zwei sich in Längsmittelrichtung der Fingerglieder von der gefrästen Bohrung aus erstreckende Schlitze von der radialen Seite aus eingefräst, die in Fig. 1 durch 21, 23 angedeutet sind. Die Form der Schlitze 21, 23 entspricht im wesentlichen der Form der Verankerungselemente 33, 34.

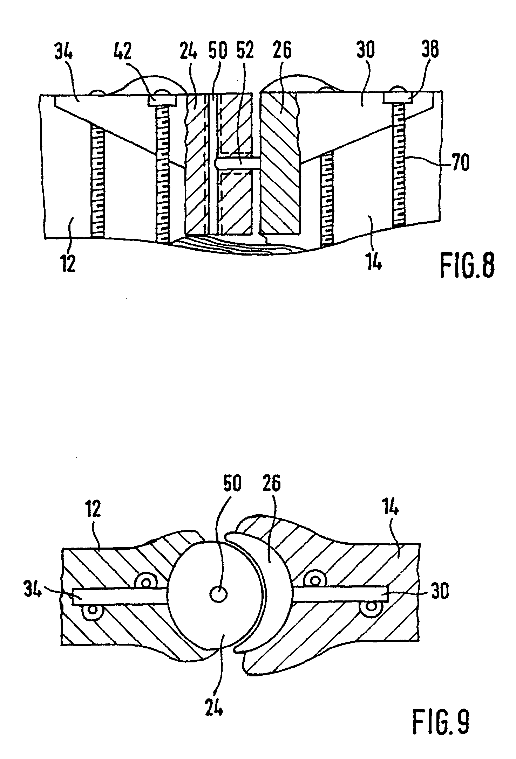

Nach dem Fräsen der Bohrung und dem Fräsen der Schlitze wird die Fingergelenkprothese 10 mit ihrem in Fig. 2 unteren Ende in die gefräste Bohrung eingeführt, wobei die Verankerungsleisten 30, 34 in die Schlitze 21, 23 eingeschoben werden, bis die Ösen 38, 40, 42 und 44 auf dem Knochen aufliegen. Anschließend werden die Verankerungsleisten 30, 34 an dem Knochenmaterial des entsprechenden Fingergliedes 12, 14 befestigt, indem Titanschrauben 70 durch die Ösen 38 bis 44 hindurch in das Knochenmaterial eingeschraubt werden, wie es in Fig. 8 gezeigt ist.

Auch die Elemente der Fingergelenkprothese sind vorzugsweise aus Titan hergestellt. Titanmaterial hat den Vorteil, daß durch das Einwachsen von Knochen in die poröse Oberfläche des Materials eine dauerhaft hohe Implantatstabilität erreicht wird.

Abhängig von der Größe des zu ersetzenden Fingergelenks liegen die Dimensionen der Fingergelenkprothese bevorzugt in folgenden Bereichen:

| | |
|---|---|
| Höhe des Gelenkkörpers | 8 bis 16 mm |
| Durchmesser des Gelenkkörpers | 4 bis 9 mm |
| Länge der Verankerungsleisten | 4 bis 8 mm |
| Dicke der Verankerungsleisten | 0,7 bis 1,3 mm |

Die Erfindung wurde anhand des Ersatzes des Fingermittelgelenks durch die Fingergelenkprothese beschrieben. Gleichermaßen kann die Fingergelenkprothese bei entsprechender Anpassung auch zu einem Ersatz der Endgelenke der Finger verwendet werden.

## Patentansprüche

1. Fingergelenkprothese mit
- einem Gelenkkörper (22), der ein erstes (24) und ein zweites Gelenkelement (26) aufweist, die schwenkbar miteinander verbunden sind und von deren Außenumfangsfläche sich jeweils ein Verankerungselement zur Befestigung an einem proximalen (12) bzw. distalen (14) Fingerglied erstreckt,
wobei
- das zweite Gelenkelement (26) bezüglich des ersten Gelenkelements (24) um die Außenumfangsfläche des ersten Gelenkelements (24) verschwenkbar ist,
- der Gelenkkörper (22) im wesentlichen zylindrisch ist, wenn sich die beiden Gelenkelemente (24, 26) in einer Implantierstellung befinden, und
- die beiden Verankerungselemente von - in Richtung der Zylinderachse gesehen - schmalen Verankerungsleisten (30, 34) gebildet werden, die sich in der Implantierstellung in einer geraden Linie in entgegengesetzte Richtungen erstrecken, und **dadurch gekennzeichnet, daß** an den Seitenwänden der Verankerungsleisten (30, 34) Ösen (38, 40, 42, 44) mit in Axialrichtung der Gelenke ausgerichteten Öffnungen (46) für die Aufnahme von Fixationsschrauben (70) angebracht sind.

2. Prothese nach Anspruch 1, **dadurch gekennzeichnet, daß** das erste (24) und das zweite Gelenkelement (26) im Querschnitt so ausgebildet sind, daß das zweite Gelenkelement (26) bei einer Bewegung aus der Implantierstellung in eine Beugestellung entlang der Außenumfangsfläche des ersten Gelenkelements (24) gleitet.

3. Prothese nach einem der vorhergehenden Ansprüche, **dadurch gekennzeichnet, daß** das zweite Gelekelement (26) im Querschnitt sichelförmig ist und das erste Gelenkelement (24) so ausgebildet ist, daß es in den konkaven Bereich des zweiten Gelenkelements (26) mit einem Teil seiner Außenumfangsfläche eingreift.

4. Prothese nach Anspruch 3, **dadurch gekennzeichnet, daß**
- an dem Mittelbereich der konkaven Fläche (28) des zweiten Gelenkelemtes (26) ein T-förmiges Achselement (51) befestigt ist, die einen Mittelsteg (52), der sich im wesentlichen senkrecht zur konkaven Fläche (28) in radialer Richtung erstreckt und eine Achse (50) aufweist, die parallel im Abstand zu dieser in Axialrichtung angeordnet ist, und
- die Achse (50) in einer in Axialrichtung des ersten Gelenkelements (24) verlaufenden Achsbohrung (48) und der Mittelsteg (52) in einem radial verlaufenden Schlitz (54) in dem ersten Gelenkelement (24) aufgenommen sind, der in die Achsbohrung (48) mündet.

5. Prothese nach Anspruch 4, **dadurch gekennzeichnet, daß** der Schlitz (54) so ausgebildet ist, daß die Achse (50) in der Implantierstellung an einer seiner in Axialrichtung verlaufenden Innenwände (66, 68) anschlägt.

## Claims

1. A finger joint prosthesis having
- a joint body (22) which has a first joint element (24) and a second joint element (26), which are pivotably connected to one another and from the outer peripheral surface of which extends a respective anchoring element for fastening to a proximal finger member (12) or distal finger member (14),
wherein
- the second joint element (26) is pivotable relative to the first joint element (24) about the outer peripheral surface of the first joint element (24),
- the joint body (22) is substantially cylindrical when the two joint elements (24,26) are situated in an implanting position, and
- the two anchoring elements are formed by (viewed in the direction of the cylinder axis) narrow anchoring bars (30,34) which in the implanting position extend in a straight line in opposite directions, and
**characterised in that** lugs (38,40,42,44) are attached to the side walls of the anchoring bars (30,34), which have axially aligned openings (46) for receiving fixing screws (70).

2. A prosthesis according to Claim 1, **characterised in that** the first joint element (24) and the second joint element (26) are so formed in cross-section that in a movement from the implanting position into a bending position the second joint element (26) slides along the outer peripheral surface of the first joint element (24).

3. A prosthesis according to either one of the preceding Claims, **characterised in that** the second joint element (26) is crescent-shaped in cross-section and the first joint element (24) is so designed that it engages into the concave region of the second joint element (26) with a part of its outer peripheral surface.

4. A prosthesis according to Claim 3, **characterised in that**
- a T-shaped axial element (51) is fastened to the medial region of the concave surface (28) of the second joint element (26), which has a medial web (52), which extends in radial direction substantially perpendicular to the concave surface (28), and an axis (50) which is disposed parallel at a distance therefrom in the axial direction, and
- the axis (50) is accommodated in an axial bore (48) extending in the axial direction of the first joint element (24) and the medial web (52) is accommodated in a radially extending slot (54) in the first joint element (24), which opens into the axial bore (48).

5. A prosthesis according to Claim 4, **characterised in that** the slot (54) is so designed that in the implanting position the axis (50) abuts against one of its inner walls (66,68) extending in an axial direction.

## Revendications

1. Prothèse d'articulation du doigt comprenant
- un corps d'articulation (22) présentant un premier élément d'articulation (24) et un deuxième élément d'articulation (26) assemblés l'un avec l'autre de manière à pouvoir pivoter et depuis la surface périphérique extérieure de chacun desquels s'étend un élément d'ancrage pour fixation à une phalange du doigt proximale (12) resp. distale (14),
- le deuxième élément d'articulation (26) pouvant pivoter autour de la surface périphérique extérieure du premier élément d'articulation (24) par rapport au premier élément d'articulation (24),
- le corps d'articulation (22) étant sensiblement cylindrique lorsque les deux éléments d'articulation (24, 26) se trouvent dans une position d'implantation et
- les deux éléments d'ancrage étant constitués, vus dans la direction de l'axe du cylindre, par d'étroites bandes d'ancrage (30, 34) qui, dans la position d'implantation, s'étendent en ligne droite dans des directions opposées, et
**caractérisée en ce que** sont placés, au niveau des parois latérales des bandes d'ancrage (30, 34), des anneaux (38, 40, 42, 44) avec des ouvertures (46) orientées axialement pour accueillir des vis de fixation (70).

2. Prothèse selon la revendication 1, **caractérisée en ce que** la section du premier élément d'articulation (24) et la section du deuxième élément d'articulation (26) sont configurées de manière telle que, lors d'un mouvement au départ de la position d'implantation pour se mettre dans une position pliée, le deuxième élément d'articulation (26) glisse le long de la surface périphérique extérieure du premier élément d'articulation (24).

3. Prothèse selon l'une des revendications précédentes, **caractérisée en ce que** le deuxième élément d'articulation (26) a une section en forme de croissant et **en ce que** le premier élément d'articulation (24) est configuré de manière telle qu'une partie de sa surface périphérique extérieure s'engage dans la zone concave du deuxième élément d'articulation (26).

4. Prothèse selon la revendication 3, **caractérisée**
- **en ce qu'**au niveau de la zone centrale de la face concave (28) du deuxième élément d'articulation (26) est fixé un élément d'axe (51) en T présentant une barrette centrale (52) qui s'étend radialement et sensiblement perpendiculairement par rapport à la face concave (28) ainsi qu'un axe (50) situé parallèlement à distance de celle-ci en direction axiale et
- **en ce que** l'axe (50) est logé dans un trou d'axe (48) orienté dans la direction axiale du premier élément d'articulation (24) et en ce que la barrette centrale (52) est logée dans une fente (54) radiale du premier élément d'articulation (24), laquelle débouche dans le trou d'axe (48).

5. Prothèse selon la revendication 4, **caractérisée en ce que** la fente (54) est configurée de manière telle que l'axe (50) s'arrête, en position d'implantation, à l'une de ses parois intérieures (66, 68) orientées axialement.
